# EUROPÄISCHE PATENTSCHRIFT

(11) **EP 0 479 857 B1**
(45) Veröffentlichungstag und Bekanntmachung des Hinweises auf die Patenterteilung: **15.12.1993**
(21) Anmeldenummer: 90910106.5
(22) Anmeldetag: 29.06.1990
(51) Int. Cl.: A61B 5/113, A61B 5/00

(54) **SENSOR FÜR VON TIERISCHEN ODER MENSCHLICHEN KÖRPERN AUSGEHENDE MECHANISCHE KRÄFTE**
SENSOR FOR MECHANICAL FORCES GENERATED BY THE ANIMAL OR HUMAN BODY
DETECTEUR DE FORCES MECANIQUES PROVENANT DU CORPS D'ANIMAUX OU D'ETRES HUMAINS

(30) Priorität: 29.06.1989 DE 8908041 U
(43) Veröffentlichungstag der Anmeldung: 15.04.1992
(73) Patentinhaber: Freiherr von Nettelhorst, Herwig, Dipl.-Ing., D-12209 Berlin (DE)
(72) Erfinder: Freiherr von Nettelhorst, Herwig, Dipl.-Ing., D-12209 Berlin (DE)
(74) Vertreter: Maikowski, Michael, Dipl.-Ing. Dr.
(86) Internationale Anmeldenummer: DE9000495
(87) Internationale Veröffentlichungsnummer: WO9100054

(56) Entgegenhaltungen:
- EP-A- 143 046
- DE-A- 3 444 635
- DE-U- 8 704 334
- FR-A- 2 459 036
- FR-A- 2 624 716

## Beschreibung

Die Erfindung betrifft einen Sensor nach dem Oberbegriff des Anspruchs 1.

Aus der DE 34 44 635 A1 ist ein Sensor bekannt, der zur Herz- , Atem- und Kreislaufüberwachung eines Patienten auf Stillstand verwendet werden kann. Dieser Sensor wird um den Thorax des Patienten geschnallt. Er weist einen druckempfindlichen Meßwertaufnehmer auf, der ein Analogsignal erzeugt. Dieses Analogsignal wird verstärkt und gleichgerichtet. Das gleichgerichtete Signal wird einem RC-Glied zugeführt, in welchem dieses Signal integriert wird. Diese Integratorschaltung bildet den zeitlichen Aplituden-Mittelwert der Ausgangssignale des Gleichrichters. Der so gebildete Amplituden-Mittelwert wird einem Komperator zugeführt dem weiterhin eine einstellbare Vergleichsspannung zugeleitet wird. Das Ausgangssignal des Komperators wird einem Frequenzgenerator zugeführt, an dessen Ausgang ein akustischer Signalgeber, wie beispielsweise ein Lautsprecher angeschlossen ist. Wird bei der Überwachung der Atmung die Atmung sehr schwach oder hört sie auf, dann ist der Amplituden-Mittelwert am Ausgang des Integrators sehr gering oder null und sinkt somit unter den eingestellten Amplituden-Vergleichswert ab, der am anderen Eingang des Komperators anliegt. Hierdurch wird ein Ausgangssignal am Ausgang des Komperators erzeugt, das den Frequenzgenerator ansteuert, wodurch dieser in Betrieb gesetzt wird. Das im Frequenzgenerator erzeugte Wechselstromsignal wird über den Warngeber als Alarmton abgestrahlt. Ein derartiger Sensor weist den Nachteil auf, daß ein akustischer Alarm auch dann ausgelöst wird, wenn Phasen unregelmäßiger aber ungefährlicher Rhythmusänderungen der Atmung auftreten. Eine sichere telemetrische Übertragung eines Alarms ist mit diesem System nicht möglich. Es wird zwar vorgeschlagen, daß der vom Warngeber abgestrahlte Warnton Frequenzen im Ultraschallbereich aufweisen kann, jedoch können diese akustischen Ultraschallschwingungen nicht in einwandfreier Weise zur telemetrischen Übertragung verwendet werden. Wenn beispielsweise ein Kleinkind über dem Sensor Kleidungsstücke trägt und mittels einer Decke zugedeckt ist. Die vom am Körper befestigten Sensor abgestrahlten Ultraschallwellen werden durch die über dem Sensor angebrachten Stoffschichten derart gedämpft, daß eine telemetrische Übetragung nicht möglich ist.

Der Erfindung liegt die Aufgabe zugrunde, einen Sensor zu schaffen, dessen Alarmauslösung derart einstell- und fixierbar ist, daß eine rasche Erkennung eines Gefahrenzustandes möglich ist, ohne daß Unregelmäßigkeiten der Frequenzen mechanischer Kräfte, die nicht durch einen gefährlichen Zustand erzeugt werden, einen Alarm auslösen.

Erfindungsgemäß wird diese Aufgabe durch die technische Lehre des Inhalts des Anspruchs 1 gelöst.

Mit Vorteil wird erfindungsgemäß ein Sensor verwendet, der Hertz'sche Wellen abstrahlt. Dieser Sender wird mittels Impulsen gleicher Impulsdauer, jedoch unterschiedlicher Pulsperiodendauer vom Meßwertaufnehmer über eine Timer-Gate-Logik angesteuert. In dieser Timer-Gate-Logik steuert der elektrische Meßwertaufnehmer über einen Signalumsetzer einen ersten Timer mit retriggerbarer Periodendauer an, dessen Ausgang mit einem Eingang eines ersten Gate verbunden ist. Die Ausgänge der beiden Gate steuern über eine ODER-Schaltung den Sender an. Ein zweiter Timer mit einer Pulsperiodendauer größer als die des ersten mit dem zweiten Eingang des ersten Gate und ein dritter Timer mit einer Pulsperiodendauer kleiner als die des ersten ist mit dem zweiten Eingang des zweiten Gate verbunden.

Mit Vorteil werden durch diese Schaltung zur Ansteuerung des Senders Impulse mit gleicher Impulsdauer in unterschiedlicher Pulsperiodendauer derart erzeugt, daß beispielsweise bei Atmung die Pulsperiodendauer größer ist als bei Nichtatmung. Auf diese Weise wird der Sender in größeren Zeitabständen bei Atmung geschaltet und gibt zur Stromeinsparung ein kurzzeitiges "Atmungssignal" ab. Die Pulsperiodendauer bei Atmung kann mit Vorteil auf einen medizinische bedingten Sicherheitswert eingestellt werden. Die kleinere Pulsperiodendauer bei Nichtatmung kann für die Auslösung eines Alarms insbesondere nach Überschreitung einer Sicherheitsgrenze verwendet werden. Insbesondere ist die Impulsdauer, d.h. die Zeit in der gesendet wird, mindestens eine Zehnerpotenz kleiner als die Impulsperiodendauer bei Atmung .

Es hat sich bei der Überwachung der Atmung insbesondere von Kleinkindern und Säuglingen zur Erkennung eines Atemstillstandes gezeigt, daß es nicht erforderlich ist das komplette Atemsignal zu übertragen, sondern nur eine Information ob ein Atemsignal vorhanden ist oder nicht. Dies ist mit der vorstehend beschriebenen Form des Sensors möglich. Die Erfahrung hat ferner gezeigt, daß es ausreicht den Zustand eines Atemstillstandes erst nach mehreren Sekunden zu erkennen, da ein Alarm erst nach einem Atemstillstand von mindestens zehn Sekunden ausgelöst werden soll.

Zur Vereinfachung der Schaltung stehen die Pulsperiodendauer des zweiten und dritten Timers und ggf. die retriggerbare Periodendauer des ersten Timers in einem ganzzahligen Verhältnisses zueinander.

Mit Vorteil ist die Impulsdauer des Ansteuerungsimpulses des Senders mindestens eine Zehnerpotenz kleiner als die Pulsperiodendauer des zweiten Timers.

Eine besonders vorteilhafte Ausbildungsform des Sensors wird dadurch gebildet, daß der Meßwertaufnehmer, der Sender und die Timer-Gate-Logik in einer biegsamen Hülle angeordnet sind, die hautverträglich ist. Der elektrische Meßwertaufnehmer kann dabei als Dehnungsmeßstreifen ausgebildet sein. Besonders vorteilhaft ist es, einen piezo-elektrischen Meßwertaufnehmer einzubauen. Besonders günstige Ergebnisse werden erzielt, wenn der elektrische Meßwertaufnehmer eine entgegengesetzt gepolte Serien-Dimorph-Platte ist.

Bei dieser Ausführungsform ist ebenfalls eine Spannungsquelle in einer biegsamen Hülle angeordnet. Insbesondere ist diese Spannungsquelle als induktiv aufladbarer Akku ausgebildet.

Ausführungsbeispiele der Erfindung sollen unter Bezugnahme auf die Figuren der Zeichung erläutert werden:

**Es zeigen:**
Fig. 1 eine schematische Darstellung eines self-containt-Sensors,
Fig. 2 ein Blockschaltbild, des in Figur 1 dargestellten Sensors und dessen Verbindung mit einem Monitor,
Fig. 3 ein Schaltbild einer Ausführungsform einer Timer-Gate-Logik
Fig. 4 eine Darstellung der mit der Schaltung gemäß Figur 4 erzeugten Signale.

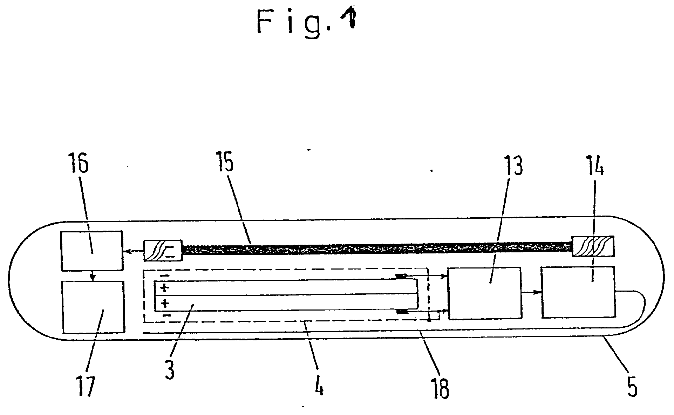

Der in Figur 1 darstellte Sensor bildet ein self-containt-System. Innerhalb einer biegsamen Hülle 5 ist außerhalb einer Abschirmung 4 ein Sender 14 angeordnet, der über eine logische Schaltung 13 mit dem Ausgang eines elektrischen Meßwertaufnehmers 3 verbunden ist. Das vom Sender 14 abgegebene Signal wird von der innerhalb der biegsamen Umhüllung 5 angeordneten Antenne 18 abgestrahlt. Dieser Sender kann ein Hochfrequenzsender oder ein induktiv arbeitender Sender sein.

Innerhalb der Hülle 5 ist ein induktiv aufladbarer Akku 17 als Spannungsquelle angeordnet. Bei 15 ist eine Ladespule schematisch dargestellt, mittels der über den Gleichrichter 16 der Akku 17 aufgeladen werden kann.

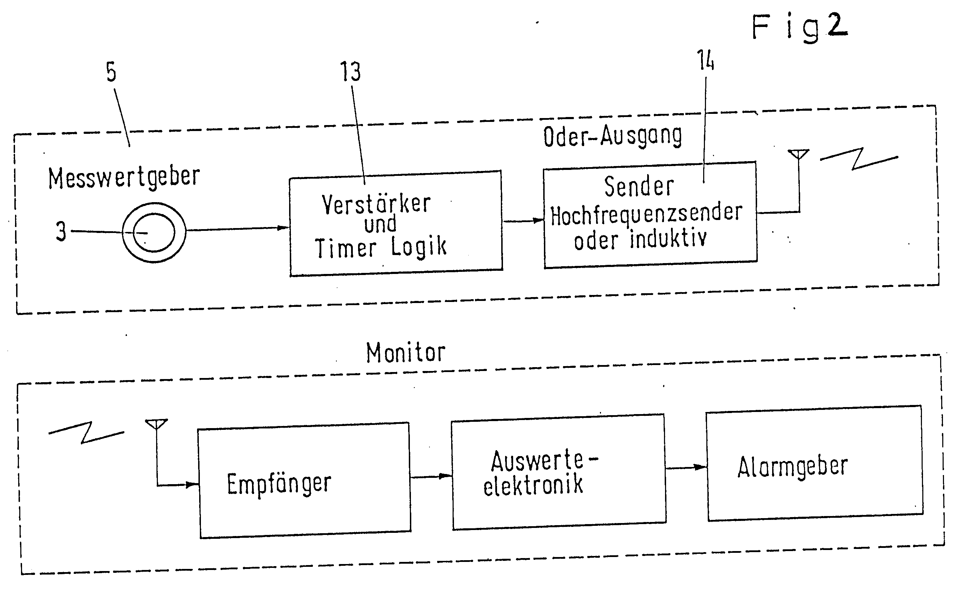

Wie schematisch in Figur 2 dargestellt, wird das vom Sender 14 abgegebene Signal in einem Monitor von einem Empfänger aufgenommen und an eine Auswerteelektronik abgegeben, die mit einem Alarmgeber verbunden ist.

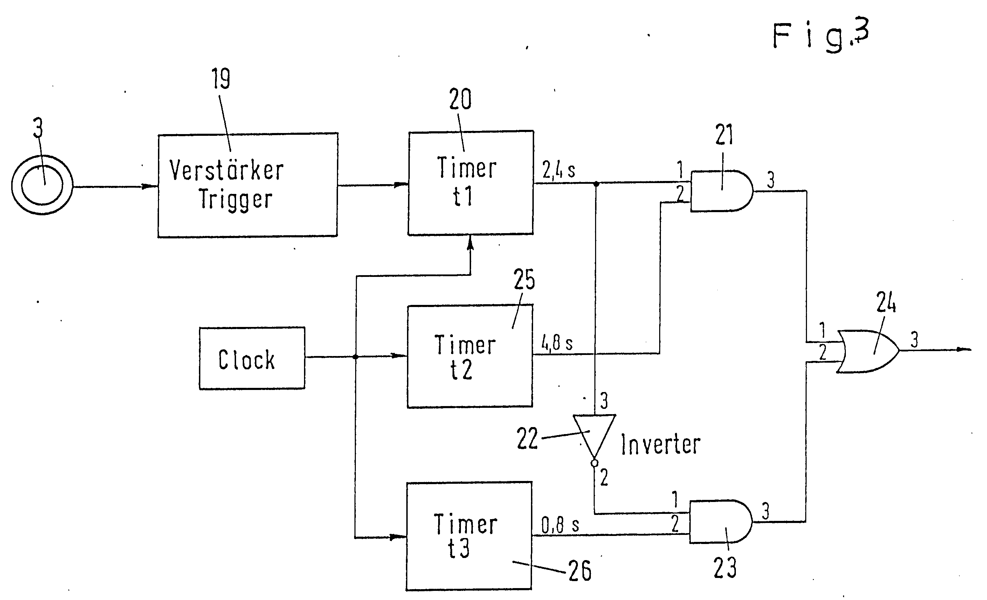

Eine vorteilhafte Ausführungsform einer Schaltung 13 ist in Figur 3 dargestellt.

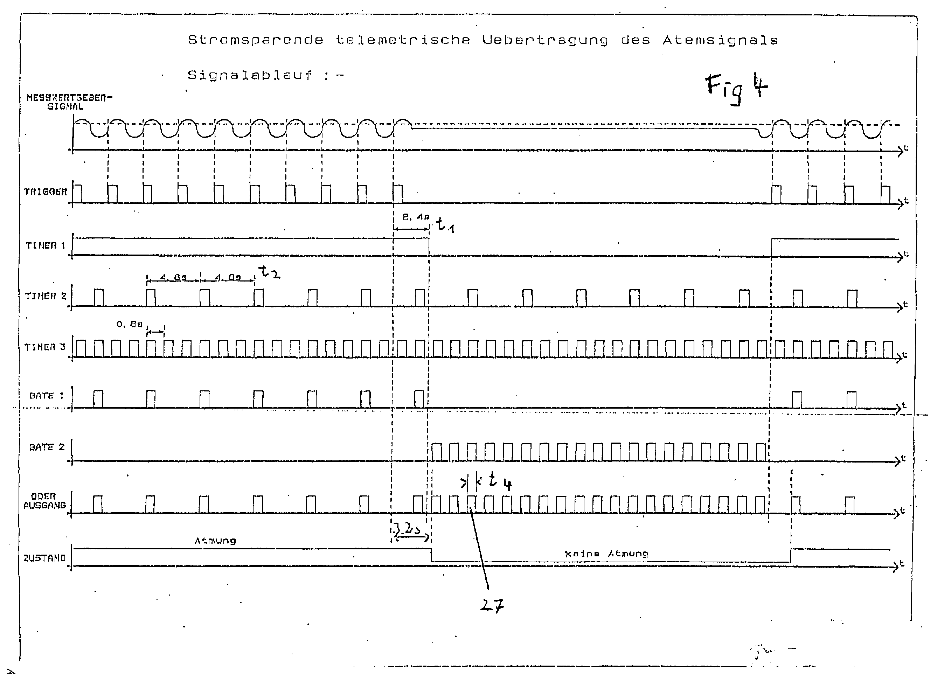

Wie Figur 3 zeigt, ist der Meßwertaufnehmer 3 mit einem Signalumsetzer 19 verbunden, der das Meßwertgebersignal in einen Signalpuls umwandelt. Dieser Signalpuls wird einem ersten Timer 20 zugeführt, der eine retriggerbare Periodendauer t1 aufweist, die beim dargestellten Ausführungsbeispiel wie Figur 4 zeigt, 2,4 s beträgt. Bei der in Figur 3 dargestellten Schaltung ist der Ausgang des ersten Timer 20 mit einem Eingang des ersten Gate 21 und über einen Inverter 22 mit einem ersten Eingang eines zweiten Gate 23 verbunden. Die Ausgänge der beiden Gate 21 und 23 steuern über die ODER-Schaltung 24 den Sender 14 an. Es ist ein zweiter Timer 25 vorgesehen, der eine Pulsperiodendauer t2 aufweist, die größer ist als die retriggerierbare Periodendauer T1 des ersten Timers 20. Bei dem dargestellten Ausführungsbeispiel beträgt diese Pulsperiodendauer, wie in Figur 5 dargestellt ist, 4,8 s. Der Ausgang dieses zweiten Timers 25 ist mit dem zweiten Eingang des ersten Gate 21 verbunden. Ein dritter Timer 26 weist eine Pulsperiodendauer t3 auf, die kleiner ist als die retriggerbare Periodendauer T1 des ersten Timers 20 und beim dargestellten Ausführungsbeispiel 0,8 s beträgt. Der Ausgang dieses dritten Timers 26 ist mit dem zweiten Eingang des zweiten Gate 23 verbunden. Diese Schaltung ermöglicht die in Figur 5 dargestellte Betriebsweise.

Bedingt durch die Atmung erzeugt der elektrische Meßwertgeber 3 Signale, die in der ersten Zeile der Figur 5 dargestellt sind. Nach einer Verstärkung werden diese Signale mittels des Signalumsetzers 19 in Rechtecksignale umgeformt, die in der zweiten Zeile der Figur 5 dargestellt sind. Diese Signale setzen den Timer 20 auf die Periodendauer t1 von beispielsweise 2,4 s. Wird innerhalb dieser 2,4 s ein weiteres Triggersignal erkannt, so wird der Timer 20 erneut auf 2,4 s gesetzt. Der Ausgang des Timers 20 hat den Pegel "High" und dieser Ausgang öffnet das Gate 21 und sperrt über den Inverter 22 das Gate 23. In diesem Betriebszustand wird vom Timer 25 über das Gate 21 jeweils nach der Periodendauer t2, vorzugsweise 4,8 s ein Signal mit einer Impulsdauer von beispielsweise 2 ms über die ODER-Schaltung 24 dem Eingang des Senders 14 zugeführt und dieser gibt ein Atemsignal von 2 ms Dauer.

Gelangt im Fall einer Atempause innerhalb von beispielsweise 2,4 s kein Triggersignal an den ersten Timer 20, so fällt der Ausgang dieses Timers 20 auf den Pegel "Low" und sperrt das Gate 21. Über den Inverter 22 wird dabei das Gate 23 geöffnet. Über dieses Gate 23 können nun vom dritten Timer 26 erzeugte Impulse mit einer Impulsperiodendauer t3 von beispielsweise 0,8 s an die ODER-Schaltung 24 gelangen, so daß der Sender 14 alle 0,8 s einen Sendeimpuls erhält. Bis zum Ende des Atemstillstandes werden nun Impulse mit einer Pulsperiodendauer von 0,8 s anstatt von 4,8 s gesendet.

Auf diese Weise läßt sich eindeutig eine Atempause erkennen. Die Signale lassen sich dadurch erkennen, daß Signale am Empfänger empfangen werden, die nicht in ein vorgegebenes Zeitraster hineinpassen.

## Patentansprüche

1. Sensor für von tierischen oder menschlichen Körpern ausgehende mechanische Kräfte mit einem elektrischen Meßwertaufnehmer, der mit einer Schaltung verbunden ist, die in Abhängigkeit von der Frequenz der mechanischen Kräfte einen Alarm auslöst,
**gekennzeichnet durch**
einen Sender (14) der mittels Impulsen (27) gleicher Impulsdauer (t4), jedoch unterschiedlicher Pulsperiodendauer (t2,t3) vom Meßwertaufnehmer (3) über eine Timer-Gate-Logik (19-26) angesteuert ist, in welcher der elektrische Meßwertaufnehmer (3) über einen Signalumsetzer (19) einen ersten Timer (20) mit retriggerbarer Periodendauer (t1) ansteuert, dessen Ausgang mit einem Eingang eines ersten Gate (21) und über einen Inverter (22) mit einem Eingang eines zweiten Gate (23) verbunden ist, die Ausgänge der beiden Gate (21,23) über eine ODER-Schaltung (24) den Sender (14) ansteuern, und ein zweiter Timer (25) mit einer Pulsperiodendauer größer als die des ersten (t2 > t1) mit dem zweiten Eingang des ersten Gate (21) und ein dritter Timer (26) mit einer Pulsperiodendauer kleiner als die der ersten (t3 < t1) mit dem zweiten Eingang des zweiten Gate (23) verbunden ist.

2. Sensor nach Anspruch 1, **dadurch gekennzeichnet,** daß die Pulsperiodendauer (t2,t3) des zweiten und dritten Timers (25,26) und gegebenenfalls die retriggerbare Periodendauer (t1) des ersten Timers in einem ganzzahligen Verhältnis zueinander stehen.

3. Sensor nach Anspruch 1 oder 2, **dadurch gekennzeichnet,** daß die Impulsdauer (t4) des Ansteuerungsimpulses (27) des Senders mindestens eine Zehnerpotenz kleiner ist, als die Pulsperiodendauer (t3) des dritten Timers (26).

4. Sensor nach mindestens einem der Ansprüche 1 bis 3, **dadurch gekennzeichnet,** daß der Meßwertaufnehmer (3), der Sender (14) und die Timer-Gate-Logik (19-26) in einer biegsamen Hülle (4) angeordnet sind.

5. Sensor nach Anspruch 4, **dadurch gekennzeichnet,** daß eine Spannungsquelle (17) in einer biegsamen Hülle (5) angeordnet ist.

6. Sensor nach Anspruch 5, **dadurch gekennzeichnet,** daß die Spannungsquelle ein induktiv (15,16) aufladbarer Akku (17) ist.

## Claims

1. Sensor for mechanical forces originating in animal or human bodies, with an electrical measured value recorder which is connected to a circuit which releases an alarm in dependence on the frequency of the mechanical forces, characterised by
a transmitter (14) which is controlled by means of impulses (27) of the same impulse length (t4) but of different pulse period length (t2, t3) from the measured value recorder (3) by way of a timer-gate logic (19-26) in which the electrical measured value recorder (3) controls by way of a signal converter (19) a first timer (20) with retriggerable period length (t1) whose output is connected to an input of a first gate (21) and by an inverter (22) with an input of a second gate (23), the outputs of the two gates (21, 23) control the transmitter (14) through an OR-circuit (24), and a second timer (25) with a pulse period length greater than that of the first (t2 > t1) is connected with the second input of the first gate (21) and a third timer (26) with a pulse period length less than that of the first (t3 < t1) is connected with the second input of the second gate (23).

2. Sensor according to claim 1 characterised in that the pulse period length (t2, t3) of the second and third timers (25, 26) and where applicable the retriggerable period length (t1) of the first timer are in a whole number ratio relative to each other.

3. Sensor according to claim 1 or 2 characterised in that the impulse length (t4) of the control impulse (27) of the transmitter is at least ten power less than the pulse period length (t3) of the third timer (26).

4. Sensor according to at least one of claims 1 to 3 characterised in that the measured value recorder (3), the transmitter (14) and the timer gate logic (19 -26) are mounted in a flexible casing (4).

5. Sensor according to claim 4 characterised in that a voltage source (17) is mounted in a flexible casing (5).

6. Sensor according to claim 5 characterised in that the voltage source is an inductively (15, 16) chargeable accumulator (17).

## Revendications

1. Capteur des forces mécaniques émises par le corps d'un animal ou de l'homme avec un enregistreur de valeurs de mesures électriques qui est relié à un couplage qui déclenche une alarme en fonction de la fréquence des forces mécaniques,
caractérisé par un émetteur (14) qui est commandé au moyen d'impulsions (27) de même durée d'impulsion (t4), toutefois de durée de période d'impulsion différente (t2, t3) par l'enregistreur de valeur de mesure (3) par l'intermédiaire d'un temporisateur-porte-logique (19-26), dans lequel l'enregistreur de valeur mesurée électrique (3) commande par l'intermédiaire d'un convertisseur de signaux (19) un premier temporisateur (20) avec une durée de période (t1) réarmable, dont la sortie est reliée avec une entrée d'une première porte (21) et par l'intermédiaire d'un inverseur (22) avec une entrée d'une deuxième porte (23), les sorties des deux portes (21, 23) commandent par l'intermédiaire d'un circuit OU (24) l'émetteur (14) et un deuxième temporisateur (25) avec une durée de période d'impulsion supérieure à celle du premier temporisateur (t2 > t1) avec la deuxième sortie de la première porte (21) et un troisième temporisateur (26) avec une durée de période d'impulsion inférieure à celle du premier temporisateur (t3 < t1) relié avec la deuxième entrée de la deuxième porte (23).

2. Capteur selon la revendication 1, caractérisé en ce que la durée de période d'impulsion (t2, t3) du deuxième et du troisième temporisateur (25, 26) et éventuellement la durée de période réarmable (t1) du premier temporisateur se situent entre elles dans une relation de nombre entier.

3. Capteur selon la revendication 1 ou 2, caractérisé en ce que la durée d'impulsion (t4) de l'impulsion de commande (27) de l'émetteur est au moins inférieure à une puissance de dix à la durée de période d'impulsion (t3) du troisième temporisateur (26).

4. Capteur selon au moins une des revendications 1 à 3, caractérisé en ce que l'enregistreur de valeur mesurée (3), l'émetteur (14) et le temporisateur-porte-logique (19, 26) sont disposés dans une enveloppe flexible (4).

5. Capteur selon la revendication 4, caractérisé en ce qu'une source de tension (17) est agencée dans une enveloppe flexible (5).

6. Capteur selon la revendication 5, caractérisé en ce que la source de tension est un accumulateur (17) rechargeable par induction (15, 16).
